# EUROPÄISCHE PATENTSCHRIFT

(11) **EP 3 536 362 B1**
(45) Veröffentlichungstag und Bekanntmachung des Hinweises auf die Patenterteilung: **01.07.2026**
(21) Anmeldenummer: 18159963.0
(22) Anmeldetag: 05.03.2018
(51) Int. Cl.: A61M 5/00, B65B 1/04, B65B 3/00, B65B 7/28

(54) **VORRICHTUNG ZUM HALTEN VON STOPFEN ÜBER EINER ÖFFNUNG EINES GEFÄSSES**
DEVICE FOR HOLDING PLUG OVER AN OPENING OF A VESSEL
DISPOSITIF DE MAINTIEN BOUCHON SUR UNE OUVERTURE D'UN RÉCIPIENT

(43) Veröffentlichungstag der Anmeldung: 11.09.2019
(73) Patentinhaber: SHL Medical AG, 6302 Zug (CH)
(72) Erfinder: Hasler, Peter, 8447 Dachsen (CH); Egloff, Christoph, 8224 Löhningen (CH); Weibel, Ludwig Daniel, verstorben (CH)

(56) Entgegenhaltungen:
- WO-A1-2016/087627
- WO-A1-2016/166769
- JP-A- H07 328 117
- US-B1- 6 290 680

## Beschreibung

Die vorliegende Erfindung betrifft eine Vorrichtung zum Halten von zumindest einem Stopfen über der Öffnung eines Gefässes sowie ein Verfahren zum Befüllen von zumindest einer Karpule gemäss den unabhängigen Patentansprüchen.

Lyophilisation oder Gefriertrocknung wird im pharmazeutischen Bereich eingesetzt, um Arzneimittel, insbesondere Wirkstoffe, die in gelöster Form nicht haltbar sind, über einen längeren Zeitraum aufzubewahren. Die Rekonstitution, d.h. das Wiederauflösen der Wirkstoffe erfolgt dabei in der Regel unmittelbar vor deren Anwendung. Gerade in jüngerer Zeit haben Arzneimittel, die auf biotechnologischem Verfahren beruhen, an Bedeutung gewonnen. Derartige Biomoleküle zeigen oft eine geringe Stabilität, insbesondere in Lösung. Entsprechend ist die Lyophilisierung von Parenteralia heutzutage ein weitverbreitetes Verfahren. Experten gehen davon aus, dass in naher Zukunft rund die Hälfte aller Injektionsmedikamente lyophilisiert wird.

In einem ersten Schritt des Gefriertrocknungsprozesses wird ein gelöster Wirkstoff direkt in ein Primärpackmittel, beispielsweise eine Phiole, eine Spritze oder eine Karpule, gegeben. Darauf wird die Lösung tiefgefroren, typischerweise bei einer Temperatur von ca. -60°C. In einem nächsten Schritt wird die gefrorene Lösung unter Vakuum gesetzt, wodurch es zu einer Sublimation ihrer flüchtigen Bestandteile, insbesondere des Lösungsmittels kommt. Als Resultat bleibt das Arzneimittel in gefriergetrockneter Form im Primärpackmittel zurück, welches zum Ende des Lyophilisationsprozesses verschlossen wird.

Gerade Karpulen sind als Primärpackmittel für gefriergetrocknete Parenteralia vorteilhaft, da diese nicht nur als Lager- und Transportbehältnis, sondern auch zur Rekonstitution und Injektion der Arzneimittel verwendet werden können. Dabei sind sowohl Einkammerkarpulen, welche lediglich das Lyophilisat aufnehmen, als auch Doppelkammerkarpulen, welche in zwei getrennten Kompartimenten sowohl ein Lyophilisat als auch ein Lösungsmittel enthalten, bekannt. Sowohl Einkammer- als auch Doppelkammerkarpulen weisen einen hohlzylindrischen Karpulenkörper auf. Dieser verjüngt sich an einem ersten Ende, welches auch als Hals bezeichnet wird, an einem dem ersten Ende gegenüberliegenden zweiten Ende hat der Karpulenkörper üblicherweise einen im Wesentlichen konstanten Durchmesser.

Zum Befüllen einer Karpule mit einem Lyophilisat ist es verbreitet, eine Lyophilisationslösung durch den Hals in die Karpule einzufüllen, wobei das dem Hals gegenüberliegende Ende des Karpulenkörpers bereits mit einem Stopfen verschlossen ist. Allerdings ist es auch bekannt, die Lyophilisationslösung durch das dem Hals gegenüberliegende weite Ende in die Karpule einzufüllen, wobei der Hals bereits verschlossen ist, beispielsweise mit einem Septum.

In diesem Zusammenhang beschreibt die WO 2012/168268 A1 einen speziellen Lyophilisationsstopfen für Karpulen. Der besagte Stopfen wird, nachdem die Karpule an dem ihrem Hals gegenüberliegenden Ende mit der Lyophilisationslösung befüllt wurde, auf dieses Ende aufgesetzt. Der Stopfen weist im Bereich seiner Unterseite mehrere sowohl in axiale Richtung nach unten als auch in radialer Richtung offene Vertiefungen auf, so dass zwischen der Wandung der Karpule und dem Stopfen ein Zwischenraum verbleibt, über welchen flüssige Bestandteile, typischerweise das Lösungsmittel, während der Lyophilisation gasförmig entweichen können. Nach erfolgter Gefriertrocknung wird der Stopfen weiter in die Karpule hineingedrückt, so dass er diese verschliesst.

Die WO 2016/087627 beschreibt eine Vorrichtung zum Verschliessen einer Spritze nach erfolgter Lyophilisation der darin befindlichen Lösung. Die Vorrichtung umfasst unter anderem einen Stopfen, eine Stopfenaufnahme, einen Spritzenträger und einen Abstandhalter. Die Elemente der Vorrichtung werden gegenseitig so ausgerichtet, dass die Spritzenöffnung während der Lyophilisation offen bleibt.

Obwohl sich die oben genannten Verfahren zum Bereitstellen von Karpulen mit gefriergetrocknetem Parenteralia bewährt haben, weisen diese Methoden noch immer den Nachteil auf, dass sie vergleichsweise zeitintensiv sind. So dauert eine Lyophilisation in der pharmazeutischen Produktion in der Regel mehrere Tage. Dies erhöht die Herstellungskosten eines Arzneimittels und limitiert die maximalen Produktionskapazitäten. Darüber hinaus sind, wie bereits erläutert, je nach Anwendungsfall spezielle Lyophilisationsstopfen erforderlich, was das Befüllen der Karpulen zusätzlich verkompliziert. Im günstigen Fall, wo handelsübliche Stopfen verwendet werden können, wird die Entgasung des Gefässes durch die Haltevorrichtung selber verlangsamt, insbesondere wenn diese Haltevorrichtung als Platte zur Verarbeitung eines Ensembles von Gefässen ausgebildet ist.

Es zeigt die JP H07 328117 ein Vakuumgefriertrocknungsverfahren und eine Vorrichtung für einen Behälter, die es ermöglichen, dass die Vakuumgefriertrocknung einer Chemikalie in einem Abschnitt des Behälters unter Verwendung eines Vakuumgefriertrocknungsverfahrens erfolgt und der Inhalt des Gefässes zusammen mit einem Trockengas im Gefäss hernach versiegelt wird. Das Verfahren ist aufwändig und beinhaltet Glasbruchsrisiken.

Es zeigt die WO2016/166769 ein Verfahren zum Verschliessen einer Vielzahl von medizinischen Kartuschen, wobei ein Verschlussnest zum Tragen einer Vielzahl von Verschlüssen verwendet wird, umfassend eine planare Trägerplatte mit einer Vielzahl von Behältern mit einer Vielzahl von rohrförmigen Behältern und Rückhaltestrukturen zum zuverlässigen Aufnehmen der Verschlüsse. Bei dem Verfahren wird die Vielzahl von medizinischen Kartuschen in einem entsprechenden Kartuschennest getragen. Das Verschlussnest ist oberhalb des Kartuschennests so angeordnet, dass die Aufnahmen des Verschlussnestes exakt mit den Aufnahmen des Kartuschennestes ausgerichtet sind. Das Verfahren ist nicht für Lyophisierschritte geeignet.

Die US 6 290 680 zeigt eine zylinderförmige Spritzenampulle mit einer am vorderen Ende angebrachten und die Öffnung abdichtenden Versiegelung und einem Kolben, der beweglich und abdichtend innerhalb des Zylinders angeordnet ist. Die Erfindung bezieht sich auf Verfahren und Mittel zur Herstellung von vorgefüllten solchen Ampullen. Das Verfahren erweist sich als aufwändig.

Es ist daher eine Aufgabe der vorliegenden Erfindung, die oben genannten Nachteile im Stand der Technik zu überwinden. Insbesondere ist es eine Aufgabe der vorliegenden Erfindung, Vorrichtungen und Verfahren zum Befüllen einer Karpule mit einem Lyophilisat zu schaffen, die einen schnelleren Gefriertrocknungsprozess ermöglichen. Darüber hinaus soll das Verfahren operationell einfach und wirtschaftlich sein. Ferner sollen die Karpulen insbesondere lediglich aus auf dem Markt erhältlichen Standardkomponenten bestehen.

Diese Aufgaben werden durch eine Vorrichtung zum Halten von wenigstens einem Stopfen gelöst, welche die Merkmale in Anspruch 1 aufweist. Die Vorrichtung zum Halten von wenigstens einem Stopfen über der Öffnung eines Gefässes umfasst zumindest eine über dem Gefäss positionierbare Halteplatte, welche mehrere Aufnahmeöffnungen für die Aufnahme des/der Stopfen aufweist. Die Aufnahmeöffnung weist mehrere Innenwandabschnitte auf, welche den Stopfen derart verschiebbar aufnehmen, dass er aus einer ersten Position über der Öffnung des Gefässes in eine zweite Position verschiebbar ist, in welcher wenigstens ein Abschnitt des Stopfens die Öffnung des Gefässes verschliesst. Die Vorrichtung sieht vor, dass die Halteplatte wenigstens eine Entgasungsöffnung aufweist, durch welche bei in die Aufnahmeöffnung eingesetztem Stopfen ein Gas von der dem Gefäss zugewandten Seite der Halteplatte auf die vom Gefäss abgewandte Seite der Halteplatte strömen kann, und, dass die Entgasungsöffnung als Schlitz zwischen wenigstens zwei Innenwandabschnitten ausgebildet ist. Die Vorrichtung ist geeignet für den Einsatz in einer Lyophilisationsapparatur.

Wie im Folgenden insbesondere in Bezug auf das erfindungsgemässe Verfahren noch näher erläutert wird, hat eine besagte Stopfenhalterung den Vorteil, dass ein gewöhnlicher Standardstopfen während der Gefriertrocknung einer in eine Karpule eingefüllten Lyophilisationslösung im Bereich des dem Hals der Karpule abgewandten Endes derselben gehalten werden kann. Dadurch, dass dasjenige Ende der Karpule, welches im Wesentlichen den Maximaldurchmesser des Karpulenkörpers hat, während des Gefriertrocknungsprozesses frei von jeglichen Hindernissen ist, können die gasförmigen flüchtigen Bestandteile der Lyophilisationslösung die Karpule ungehindert verlassen. Die Lyophilisationszeiten können damit deutlich reduziert werden. Darüber hinaus entfällt der Einsatz von speziell angepassten Lyophilisationsstopfen, wodurch die Karpule aus günstigen Standardkomponenten aufgebaut werden kann.

Es versteht sich allerdings von selbst, dass die Verwendung einer derartigen Stopfenhalterung nicht auf den Einsatz in einer Lyophilisationsapparatur beschränkt ist.

Vorzugsweise umfasst die Halteplatte mehrere Stopfenaufnahmen, mit denen jeweils ein Stopfen haltbar ist. Bevorzugterweise sind die Stopfenaufnahmen nebeneinander angeordnet. Mit einer entsprechenden Halteplatte können mehrere Karpulen gleichzeitig prozessiert werden. Besonders bevorzugt ist ein Lochbild, welches an die branchenübliche Anzahl und Anordnung von Karpulen angepasst ist. Bevorzugt bildet das Lochbild der Halteplatte die Anordnung von Karpulenablagekörben ab, wie sie standardmässig von Karpulenzulieferern (wie z.B. Ompipharma oder Schott) verwendet werden. Dadurch kann ein Standard-Ensemble von Karpulen auf effiziente Weise befüllt, lyophilisiert und verschlossen werden.

Die Halteplatte umfasst wenigstens eine Entgasungsöffnung, die bei in die Stopfenaufnahme eingesetztem Stopfen von einem Gas passierbar ist. Genauer kann ein Gas von der dem Gefäss zugewandten Seite der Halteplatte auf die dem Gefäss abgewandte Seite der Halteplatte strömen. Dadurch können flüchtige Bestandteile während des Lyophilisationsprozesses die Halteplatte leicht passieren, was den Materialstrom der flüchtigen Bestandteile innerhalb der Lyophilisationsapparatur weiter begünstigt und damit die für die Gefriertrocknung erforderliche Zeitdauer verkürzt.

Erfindungsgemäss weist die Halteplatte mehrere Öffnungen auf. So kann für den Fall, dass die Halteplatte mehrere Stopfenaufnahmen aufweist, jede Stopfenaufnahme eine Durchtrittsöffnung aufweisen. Allerdings ist es auch möglich, ja sogar bevorzugt, dass pro Stopfenaufnahme mehrere Entgasungsöffnungen an der Halteplatte angeordnet sind.

Die Entgasungsöffnungen sind als Schlitze zwischen den Innenwandabschnitten ausgebildet sein. Dadurch sind die Öffnungen unmittelbar im Bereich einer Stopfenaufnahme angeordnet, was den Materialstrom innerhalb der Lyophilisationsvorrichtung weiter begünstigt. Darüber hinaus kann durch diese Positionierung der Entgasungsöffnungen die Ausgestaltung von Griffbacken leichter realisiert werden.

Die Innenwandabschnitte können also eine unterbrochene Haltefläche bilden, wobei jeder Teil der Haltefläche die Form eines Kreisbogens hat. Der Radius dieses Kreisbogens entspricht vorzugsweise dem Radius des Stopfens. Allerdings kann die Haltefläche auch aus geraden Teilen bestehen.

Die Innenwandabschnitte können insbesondere eine unterbrochene, kegelstumpfförmige Haltefläche bilden, mit welcher der Stopfen in Kontakt bringbar und haltbar ist. Durch die konische Form der Haltefläche kann der Stopfen leichter in die Stopfenaufnahme eingeführt werden.

Nachfolgend wird unter der Oberseite der Halteplatte diejenige Seite verstanden, gegen welche sich die kegelstumpfförmige Haltefläche aufweitet.

Die Stopfenaufnahme kann 2, 3, 4, 5, 6, 7 oder 8 Innenwandabschnitte umfassen. Vorteilhaft sind in diesem Zusammenhang insbesondere 3 bis 6 Innenwandabschnitte. Damit kann der Stopfen leicht in die Stopfenaufnahme eingeführt und auch wieder aus diesem entfernt werden.

In einer bevorzugten Ausführungsform sind die Innenwandabschnitte, welche Bestandteil der Aufnahmeöffnung sein können, durch wenigstens zwei Griffbacken gebildet. Zwischen diesen Griffbacken ist der Stopfen haltbar.

Die Griffbacken können federelastisch sein. Dies ermöglicht ein sicheres Halten des Stopfens in der entsprechenden Stopfenaufnahme, wobei nicht ausschliesslich auf die Eigenelastizität des Stopfens Verlass sein muss. Darüber hinaus gehen die Griffbacken bei einer solchen Ausgestaltung der Halteplatte nach deren Gebrauch selbständig wieder zurück in ihre Ausgangsposition, wodurch eine derartige Halteplatte zur mehrfachen Verwendung geeignet ist.

Die Halteplatte umfasst einen umlaufenden Stützkragen umfassen, mit welchem die Halteplatte insbesondere in horizontaler Lage haltbar ist. Der besagte Stützkragen ist an einer der Oberseite der Halteplatte gegenüberliegenden Unterseite angeordnet. Ein derartiger Stützkragen hat den Vorteil, dass die Halteplatte während des Gefriertrocknungsprozesses nicht auf der Karpule aufliegt. Dadurch kann verhindert werden, dass fragiles Karpulenmaterial, z.B. Glas, einem Druck durch die aufliegende Halteplatte ausgesetzt ist und dadurch Schaden nimmt. Insbesondere können somit Karpulen verwendet werden, die weitaus grössere Fertigungstoleranzen aufweisen.

Die Halteplatte und der die Halteplatte umlaufende Stützkragen können aus Kunststoffmaterial, vorzugsweise aus einem nach GMP autoklavierbaren, strahlensterilisierbaren oder plasmasterilisierbaren Kunststoffmaterial gebildet sein. Der Stützkragen kann ausserdem eine strahlenreflektierende Oberfläche aufweisen. Damit wird bei der gleichzeitigen Verarbeitung eines Ensembles von Karpulen der von der Lage des einzelnen Gefässes abhängige Einfluss der Umgebungsstrahlung reduziert. Ein solcher Abschirmungseffekt kann zusätzlich oder alternativ zum Abschirmungseffekt einer bevorzugt metallischen Sockelplatte wirken, wie es weiter unten beschrieben wird.

Die Halteplatte kann zumindest eine Zentrierstruktur zum Zentrieren von zumindest einer Stopfenaufnahme gegenüber einer Karpule aufweisen. Durch eine derartige Zentrierstruktur ist sichergestellt, dass der Stopfen nach erfolgter Gefriertrocknung direkt aus der Stopfenaufnahme in die Karpule eingeführt werden kann.

Die Zentrierstruktur kann als um die Stopfenaufnahme angeordnete Vorsprünge ausgebildet sein. Dadurch erfolgt eine Zentrierung direkt an der Karpule. Die Vorsprünge können als zur Oberseite hin zulaufende Kegelsegmente ausgebildet, aufweisend einen Anzug von ca. 1 bis 2 Grad.

Allerdings kann die Zentrierstruktur alternativ oder zusätzlich am Stützkragen ausgebildet sein. Insbesondere kann die Zentrierstruktur an der Unterseite des Stützkragens ausgebildet sein. Wie im Nachfolgenden noch weiter erläutert wird, erfolgt die Zentrierung damit indirekt über eine Sockelplatte.

Die Halteplatte kann einstückig, insbesondere aus einem Kunststoffmaterial, vorzugsweise aus einem nach GMP autoklavierbaren, strahlensterilisierbaren oder plasmasterilisierbaren Kunststoffmateraial, ausgeführt sein. Kunststoffmaterialien sind besonders gut zur Verarbeitung mittels Spritzguss geeignet. Entsprechend lassen sich derartige Halteplatten kostengünstig in hohen Stückzahlen herstellen.

Die Halteplatte kann zum Halten von mehreren Stopfen ausgeführt sein.

Die Vorrichtung umfasst weiter wenigstens ein Druckausgleichselement umfassen, welches durch die Aufnahmeöffnung hindurch in die Karpule einbringbar ist. Das so eingebrachte Druckausgleichselement ragt zwischen dem Stopfen und der Karpulenwand in die Karpule hinein. Dadurch kann der Stopfen nach erfolgter Lyophilisierung entlang der Gefässwand in die Karpule hinein verschoben werden, ohne dass die Karpule durch den Stopfen hermetisch abgedichtet wird. Dadurch wird verhindert, dass mit der Volumenreduktion ein im Vergleich zum Umgebungsdruck erhöhter Druck im Innern der Karpule aufgebaut wird. Im Gegenteil ist durch geeignete Wahl der Reihenfolge von Entfernen der Druckausgleichselemente einerseits und (Schutzgas-)Belüften der Lyophilisationsapparatur andererseits wahlweise ein Unterdruck oder ein Umgebungsdruck in der fertig verschlossenen Karpule einstellbar.

Insgesamt kann also durch die Einschubtiefe des Stopfens, durch Einsatz oder Verzicht auf Druckausgleichselemente und durch Wahl des Belüftungszeitpunktes der Lyphilisationsapparatur der in der fertig verschlossenen Karpule resultierende Druck über ein Spektrum von Überdruck zu Atmosphärendruck und weiter zu Untertruck präzise eingestellt werden.

Das wenigstens eine Druckausgleichselement ist bevozugt aus einem flexiblen Kunststoffmaterial gefertigt. Der in die Karpule hineinragende Teil des Druckausgleichselements ist bevorzugterweise dünn, d.h. weist bei der Verwendung in einer Standard-Karpule bevorzugt etwa 0.5 bis 2 mm Durchmesser auf, und weist eine glatte Oberfläche auf. Diese Eigenschaften gewährleisten gute Entfernbarkeit des Druckausgleichselements, ohne dass durch die entstehende Reibung die Position des Stopfens verändert würde. Das Druckausgleichselement ist dabei so ausgebildet, dass es mehrere Beine aufweist, von denen jedes gleichzeitig in eine andere Karpule hineinragt.

In einer bevorzugten Ausführungsform umfasst die Haltvorrichtung weiter eine Stiftplatte, so beschaffen, dass sie auf die Halteplatte aufsetzbar ist, sodass auf den wenigstens einen Stopfen jeweils das Ende eines Stifts zu liegen kommt. Die Stiftplatte dient dazu, die in den Aufnahmeöffnungen der Halteplatte befindlichen Stopfen nach Abschluss der Lyophilisation von der ersten Position über der Öffnung des Gefässes in die zweite Position innerhalb des Gefässes zu verschieben, wobei der Stopfen in der zweiten Position ganz oder teilweise innerhalb des Gefässes positioniert sein kann.

Der Abschnitt eines Stopfens kann zwar auch mit einer Beaufschlagplatte in die Gefässöffnung eingeführt werden, etwa indem mit der Beaufschlagplatte auf die Oberseite der Halteplatte gedrückt wird und herausstehende Stopfen damit in Richtung der Gefässe bewegt werden. Das vollständige Einführen eines Stopfens in die Karpule ist jedoch mittels Stiftplatte besser möglich.

Die Stiftplatte hat den Vorteil, dass der oder die Stopfen genau in einer vordefinierten Position innerhalb der Karpule zu liegen kommen. Dies gilt insbesondere dann, wenn die Stiftplatte zusammen mit Druckausgleichselementen eingesetzt wird und die Druckausgleichselemente erst nach Wiedereinstellen des Atmosphärendrucks entfernt werden. Die Position der Stopfen wird dann nicht nachträglich während Druckausgleichprozessen nochmals verändert.

Dadurch wird eine gleichmässige Positionierung des Stopfens in der fertig befüllten Karpule optimal gewährleistet.

Es ist ein weiterer Vorteil der Verwendung der Stiftplatte und insbesondere der Verwendung der Stiftplatte zusammen mit Druckausgleichselementen, dass eine Einwirkung auf den Lyocake verhindert werden kann. Denn wenn der Stopfen unter Erzeugung eines Überdrucks dichtend in die Karpule eingeschoben wird oder wenn der Stopfen zufolge eines Druckunterschieds zwischen dem Innendruck der Karpule und dem äusserem Atmosphärendruck verschoben wird, kann dies den Lyocake komprimieren oder auf andere Weise dessen Aufbau in unerwünschter Weise beeinflussen.

In einer bevorzugten Ausführungsform weist die auf die Halteplatte aufsetzbare Stiftplatte ebenfalls Entgasungsöffnungen auf. Diese können als runde Öffnungen jeweils zwischen den Stiften vorliegen. Wie die Entgasungsöffnungen in der Halteplatte, dienen auch die Entgasungsöffnungen der Stiftplatte der effizienten und raschen Lyophilisierung des Karpuleninhalts.

In einer besonders bevorzugten Ausführungsform sind die Entgasungsöffnungen der Stiftplatte im Zentrum grösser als in Randnähe. Diese Ausführungsform trägt dem Umstand Rechnung, dass die Umgebungsstrahlung, trotz Abschirmungswirkung von Sockelplatte und/oder Stützkragen, besonders auf die am äusseren Rand eines Ensembles angeordneten Gefässe einwirkt, weil diese die im Innern des Ensembles angeordneten Gefässe abschirmen. Durch die grösseren Öffnungen wird der Materialstrom der flüchtigen Bestandteile bei den im Innern des Ensembles angeordneten Karpulen im Vergleich zu den peripheren Karpulen begünstigt. Die für die Gefriertrocknung erforderliche Zeitdauer über alle Gefässe des Ensembles hinweg wird nivelliert. Unterschiedliche Lyophilisierungsgeschwindigkeiten sind nicht wünschenswert, weil sie die lückenlose Kontrolle des optimalen Standardherstellungsprozesses erschweren.

Die erfindungsgemässe Vorrichtung kann weiter eine Sockelplatte mit Aufnahmeöffnungen für wenigstens ein Gefäss, insbesondere wenigstens eine Karpule umfassen. Die Sockelplatte kann zum Halten von mehreren Karpulen ausgebildet sein. Besonders bevorzugt ist ein Lochbild, welches an die branchenübliche Anzahl und Anordnung von Karpulen angepasst ist. Bevorzugt bildet das Lochbild der Halteplatte die Anordnung von Karpulenablagekörben ab, wie sie standardmässig von Karpulenzulieferern (wie z.B. Ompipharma oder Schott) verwendet werden. Dadurch kann ein Standard-Ensemble von Karpulen auf einfache und effiziente Weise in die Sockelplatte eingesetzt werden.

Die Sockelplatte kann insbesondere einstückig, vorzugsweise aus einem Metall, ausgeführt sein. Eine Ausführung aus Metall ist bei einer derartigen Sockelplatte vorteilhaft, da Metall eine bessere Temperaturleitfähigkeit als beispielsweise Kunststoffmaterialien besitzt, wodurch ein besserer Temperaturtransfer während der Lyophilisation erzielt werden kann. Auch damit kann die zur Lyophilisation erforderliche Zeit reduziert werden. Ausserdem wird das Material bevorzugt so gewählt, dass Umgebungsstrahlung abgeschirmt wird. Umgebungsstrahlung ist deshalb abzuschirmen, weil sie besonders auf die am äusseren Rand des Ensembles angeordneten Gefässe einwirkt. Diese äusseren Gefässe und deren Inhalt würden ohne Schutz einen grösseren Anteil der Umgebungsstrahlung absorbieren als die im Innern des Ensembles angeordneten Gefässe. Dadurch bestünde die Gefahr, dass die Lyophilisierung in den einzelnen Gefässen mit unterschiedlicher Geschwindigkeit abliefe. Solche Unterschiede sind nicht wünschenswert, weil sie die lückenlose Kontrolle des StandardHerstellungsprozesses erschweren. Um die Abschirmung der zu lyophilisierenden Lösung während des Vorgangs zu optimieren, können die Aufnahmeöffnungen der Sockelplatte so tief ausgebildet sein, dass sie die Karpulen wenigstens bis zur typischen Füllhöhe der verwendeten Karpulen aufnehmen. Als besonders geeignet hat sich die Verwendung von Stahl oder Titan für die Herstellung der Sockelplatte erwiesen.

In einer bevorzugten Ausführungform umfasst die Vorrichtung ausserdem ein komplementäres Positioniermittel, mit welchem die Halteplatte und die Sockelplatte, gegebenenfalls zusätzlich auch eine Stiftplatte gegenseitig positionierbar sind. Das Positioniermittel erleichtert das Assemblieren von Sockelplatte, Halteplatte und Stiftplatte. Sie reduzieren die Gefahr, dass Glaskarpulen beschädigt werden und verleihen der Vorrichtung Stabilität.

Insbesondere können in der Sockelplatte Aufnahmevertiefungen für die Aufnahme von an der Halteplatte angebrachten Positionierungsbolzen eingelassen sein. Diese sind bevorzugt als Langlöcher ausgebildet, um Spiel für Verschiebungen zuzulassen, welche sich aufgrund unterschiedlicher Ausdehnungskoeffizienten der verwendeten Materialien ergeben können.

Ein weiterer Aspekt der vorliegenden Erfindung betrifft ein Set umfassend eine Halteplatte wie vorgängig beschrieben und zumindest einen Stopfen, wobei in zumindest eine Stopfenaufnahme, insbesondere gegebenenfalls in jede Stopfenaufnahme, ein Stopfen ganz oder teilweise eingesetzt ist.

Beim Stopfen handelt es sich in der Regel um einen solchen aus einem in der pharmazeutischen Industrie üblichen Elastomer. Allerdings können je nach Anwendung prinzipiell auch Glasstopfen eingesetzt werden.

Ein derartiges Set hat den Vorteil, dass es bereits vormontiert einem pharmazeutischen Betrieb geliefert werden kann und, wie im Folgenden noch erläutert wird, insbesondere nur noch in eine Lyophilisationsapparatur eingelegt werden muss.

Das Set kann zusätzlich eine Stiftplatte, eine Sockelplatte und/oder mindestens ein Druckausgleichselement umfassen. Es ist bevorzugt, dass die Druckausgleichselemente, wie die Stopfen, bereits in die Sattelvertiefungen eingebracht sind, so dass ihre Beine durch die Aufnahmeöffnung hindurch auf die dem Gefäss zugewandte Seite (Unterseite) ragen. Durch möglichst vollständige Assemblierung aller Elemente des Sets vor dem Verpacken verringert sich der spätere Manipulationsbedarf beim Einbringen des Sets in die Lyophilisationsapperatur oder während das Set sich bereits in der Lyophilisationsapparatur befindet.

Wenn das Set eine Stiftplatte enthält, enthält es bevorzugt auch eine Transportsicherung. Unter einer Transportsicherung wird hier ein zwischen der Stiftplatte und der Stopfenaufnahme angebrachtes Abstandhaltemittel verstanden, welches in einem assemblierten Set verhindert, dass die Stiftplatte zufolge Schwerkraft oder anderer Kräfte während des Transports vorzeitig den oder die Stopfen zu weit in die Stopfenaufnahme hinein bewegt. Unter einem assemblierten Set ist in diesem Zusammenhang der einsatzbereite Stapel aus wenigstens der Stopfenaufnahme, einem oder mehreren Stopfen, Transportsicherung und Stiftplatte gemeint.

Das Abstandhaltemittel kann bevorzugt an der Stiftplatte, besonders bevorzugt an einem an der Stiftplatte ausgebildeten Positioniermittel befestigt werden. Das Abstandhaltemittel kann als Klammer ausgebildet sein, deren zwei Klemmglieder dank einem Federelement kraftschlüssig das Positioniermittel umfassen. Die Ausführung als Klemmen gewährleistet einerseits zuverlässigen Halt zwischen der Stiftplatte und der Stopfenhalterung. Andererseits ist eine Klemme auch besonders gut handhabbar. Die Klammer lässt sich leicht Anbringen, leicht horizontal Ausdrehen, zum Beispiel von einer mit dem äusseren Rand der Stiftplatte bündigen Transportposition in eine von den assemblierten Platten wegstehenden Position. Die von den assemblierten Platten wegstehende Position erleichtert das Entfernen der Klemme, etwa beim Arbeiten in einer Glove-Box.

Ein derartiges Set kann ausserdem ein Packmittel umfassen, in welches die Halteplatte mit dem zumindest einen Stopfen eingepackt ist. Die Halteplatte und die Stopfen können innerhalb des Packmittels vorsterilisiert bereitgestellt werden. Das Packmittel kann entsprechend die Halteplatte und den zumindest einen Stopfen hermetisch umschliessen. Das Packmittel kann eine Folie umfassen.

Selbstverständlich können auch die übrigen Elemente der Vorrichtung und des Sets innerhalb des Packmittels vorsterilisiert bereitgestellt werden. Neben der Halteplatte und dem Stopfen können zusätzlich auch eine Stiftplatte und/oder wenigstens ein Druckausgleichselement und/oder eine Sockelplatte und/oder Abstandhalteelement in dem Packmittel vorsterilisiert bereitgestellt werden. In einer bevorzugten Ausführungsform wird ein einsatzbereiter Stapel aus Sockelplatte, Stopfenhalteplatte, einem oder mehreren Stopfen, Abstandhalteelementen und Stiftplatte (von unten nach oben) in dem Packmittel vorsterilisiert bereitgestellt. Damit ist das Set während des Transports stapelbar und das Handling unter sterilen Bedingungen wird vereinfacht.

Ein weiterer Aspekt der vorliegenden Erfindung betrifft ein Verfahren zum Befüllen von zumindest einer Karpule, insbesondere mehrerer Karpulen, mit einem Lyophilisat, umfassend die folgenden Schritte:
- Bereitstellen der zumindest einen Karpule, wobei die Karpule von einer Sockelplatte gehalten ist;
- Befüllen der zumindest einen Karpule mit einer Lyophilisationslösung;
- Bereitstellen von zumindest einem Stopfen, wobei der Stopfen mit einer Halteplatte, insbesondere einer Halteplatte wie oben beschrieben, gehalten ist;
- Positionieren des zumindest einen Stopfens mittels der Halteplatte im Bereich einer Öffnung der Karpule;
- Lyophilisieren der Lyophilisationslösung mittels einer Lyophilisationsapparatur, um ein Lyophilisat zu erhalten;
- Einsetzen des zumindest einen Stopfens in die zumindest eine Karpule.

Das besagte Verfahren ermöglicht es, den Lyophilisationsprozess signifikant zu beschleunigen. Da der Stopfen während der Lyophilisation lediglich im Bereich der Öffnung der Karpule gehalten wird, ist diese im Wesentlichen frei von Hindernissen. Entsprechend kann innerhalb der Lyophilisationsapparatur ein optimierter Massenstrom erzielt werden. Die flüchtigen Bestandteile der Lyophilisationslösung können die Karpule entsprechend leichter verlassen, wodurch die Gesamtdauer des Gefriertrocknungsprozesses erheblich reduziert wird. Der Stopfen ist dennoch im Bereich der Öffnung der Karpule gehalten und lässt sich nach erfolgter Lyophilisation leicht in diese einführen. Darüber hinaus können mit einem entsprechenden Verfahren handelsübliche Stopfen verwendet werden. Der Einsatz von speziellen Lyophilisationsstopfen entfällt.

Bei einem derartigen Verfahren kann die Karpule durch die Öffnung an dem dem Hals gegenüberliegenden Ende der Karpule mit der Lyophilisationslösung befüllt werden. Damit kann die Öffnung sowohl zum Befüllen der Karpule mit Flüssigkeit als auch zum Abziehen der flüchtigen Bestandteile während der Lyophilisation verwendet werden. Der Hals der Karpule kann, insbesondere mit einem Septum, verschlossen sein, und die Öffnung durch das dem Hals gegenüberliegende Ende der Karpule gebildet sein.

Dadurch, dass die dem Hals gegenüberliegende Öffnung der Karpule einen Durchmesser aufweist, welcher im Wesentlichen dem maximalen Gesamtdurchmesser der Karpule entspricht, kann die erforderliche Zeitdauer für die Lyophilisation in einem besonders grossen Ausmass reduziert werden.

Der zumindest eine Stopfen kann oberhalb der Öffnung der Karpule gehalten sein. Dadurch lässt sich der Stopfen unmittelbar nach Beendigung der Gefriertrocknung besonders einfach in die Karpule einführen.

Das Einführen des Stopfens in die Karpule kann durch Beaufschlagen des Stopfens mit einer Kraft von der der Karpule abgewandten Seite erfolgen. Das Beaufschlagen des Stopfens kann insbesondere durch eine in der Lyophilisationsapparatur angeordnete Beaufschlagplatte erfolgen. Damit kann die zumindest eine Karpule noch innerhalb der Lyophilisationsapparatur verschlossen werden.

Das Beaufschlagen des Stopfens kann alternativ durch eine oberhalb der Halteplatte angeordnete Stiftplatte erfolgen. Der Einsatz einer Stiftplatte bietet den Vorteil, dass der Stopfen beim Einführen in die Karpule auf eine bestimmte gewünschte Position innerhalb des Karpulenkörpers gebracht werden kann. Es kann dadurch sichergestellt werden, dass der Stopfen vollständig, d.h. mit allen optional am Stopfen ausgebildeten Dichtlippen, in das Gefäss eingeführt wird und die Dichtungswirkung des Stopfens somit erhöht wird.

Das Einsetzen des Stopfens in die Karpule kann bei einem Druck geringer als Atmosphärendruck erfolgen. In diesem Zusammenhang kann das erfindungsgemässe Verfahren zusätzlich den folgenden Schritt umfassen: Belüften der Lyophilisationsapparatur auf Atmosphärendruck.

Dabei kann der Stopfen beim Belüften der Lyophilisationsapparatur auf Atmosphärendruck entlang der Längsrichtung der Karpule verschoben werden. Dies kann insbesondere durch Eigenwirkung des Atmosphärendruckes auf den Stopfen erfolgen. Die luftdruckgetriebene Positionierung des Stopfens kann jedoch zu Gefässschäden, insbesondere zu Glasbruch, führen. Ausserdem kann die Endposition des Stopfens aufgrund unterschiedlicher Reibung zwischen den einzelnen Stopfen und Gefässen variieren. Solche Variationen erschweren die lückenlose Kontrolle der optimalen Herstellungsbedingungen, weshalb die Positionierung via Stiftplatte bevorzugt ist.

Das erfindungsgemässe Verfahren kann daher auch die folgenden Schritte umfassen:
- Einsetzen eines Druckausgleichselementes in die Karpule, vor dem Einsetzen des Stopfens in die Karpule;
- Entfernen des Druckausgleichselementes aus der Karpule, nach dem Einsetzen des Stopfens in die Karpule.

Durch den Einsatz eines derartigen Druckausgleichselementes kann der in der Karpule resultierende Druck nach Bedarf eingestellt werden. Ein während der Lyophilisierung herrschender Unterdruck kann beibehalten werden, indem die Stopfen noch in Unterdruckatmosphäre in Position gebracht und das Druckausgleichselement anschliessend entfernt wird. Die Karpule kann aber auch vor dem Entfernen der Druckausgleichselemente belüftet werden, etwa mit Schutzgas, wobei in der fertig verschlossenen Karpule Umgebungsdruck resultiert. Wenn nach dem Verschliessen der Karpule in dieser kein Unterdruck mehr besteht, verringert sich das Risiko, dass während der Lagerung Feuchtigkeit oder Sauerstoff in das Innere der Karpule gelangen könnten. Dies ist insbesondere von Interesse, wenn die Lyophilisationsapparatur nach erfolgter Gefriertrocknung mit einem Schutzgas, bspw. Argon oder Stickstoff, befüllt wird.

Bei in die Sockelplatte eingelegter Karpule kann die Öffnung bei bestimmungsgemässem Gebrauch der Sockelplatte, insbesondere lotrecht, nach oben ausgerichtet sein. Eine Karpulenhalterung, mit der mehrere Karpulen gleichzeitig haltbar sind, ermöglicht das parallele Befüllen von mehreren Karpulen.

Es versteht sich von selbst, dass die Erfindung nicht auf die beschriebenen Ausführungsbeispiele beschränkt ist. Die Vorrichtungsbestandteile können so ausgestaltet sein, dass verschiedene handelsübliche Stopfen (Dättwilerstopfen, Weststopfen) und Gefässe (Cartridges, Vials) prozessierbar sind. Ebenso kann die Vorrichtung modular aufgebaut sein, sodass durch Kombination unterschiedlich ausgebildeter Bestandteile optimale Bedingungen für verschiedene Füllstandshöhen, Druckverhältnisse und Lyophilisatorgeräte hergestellt werden können.

Weitere Vorteile und Einzelmerkmale der Erfindung ergeben sich aus der nachfolgenden Beschreibung zweier Ausführungsbeispiele und aus den Zeichnungen.

Es zeigen schematisch:
- Figur 1:: Explosionsansicht einer Lyophilisationsanordnung umfassend eine erfindungsgemässe Halteplatte und Stiftplatte;
- Figur 2:: Perspektivische Darstellung der Ausführungsform mit Stiftplatte in einer ersten Position;
- Figur 3:: Perspektivische Darstellung der Ausführungsform mit Stiftplatte in einer zweiten Position;
- Figur 4:: Perspektivische Teilvergrösserung der Halteplatteoberfläche mit aufgenommenen Stopfen und ganz und teilweise eingebrachten Druckausgleichselementen;
- Figur 5:: Perspektivische Darstellung der Ausführungsform mit Beaufschlagplatte in einer ersten Position;
- Figur 6:: Perspektivische Teilvergrösserung der Halteplatteoberfläche mit aufgenommenen Stopfen in einer ersten Position;
- Figur 7 und 8:: Schrittweise Darstellung eines erfindungsgemässen Verfahrens zum Verschieben des Stopfens aus einer ersten Position über der Öffnung in eine zweite Position, in welcher wenigstens ein **Ab-** schnitt des Stopfens die Öffnung des Gefässes verschliesst.
- Figur 9:: Draufsicht auf eine erfindungsgemässe Halteplatte;
- Figur 10:: Ansicht von unten auf eine erfindungsgemässe Halteplatte;
- Figuren 11 bis 14:: Perspektivische Darstellungen des erfindungsgemässen Verfahrens in vier sequenziellen Assemblierungsschritten;
- Figuren 15 bis 16:: Perspektivische Darstellung eines erfindungsgemässen Sets (Stopfen und Druckausgleichselemente nicht gezeigt) zusätzlich umfassend zwei Abstandhaltemittel, vor und nach der Assemblierung.

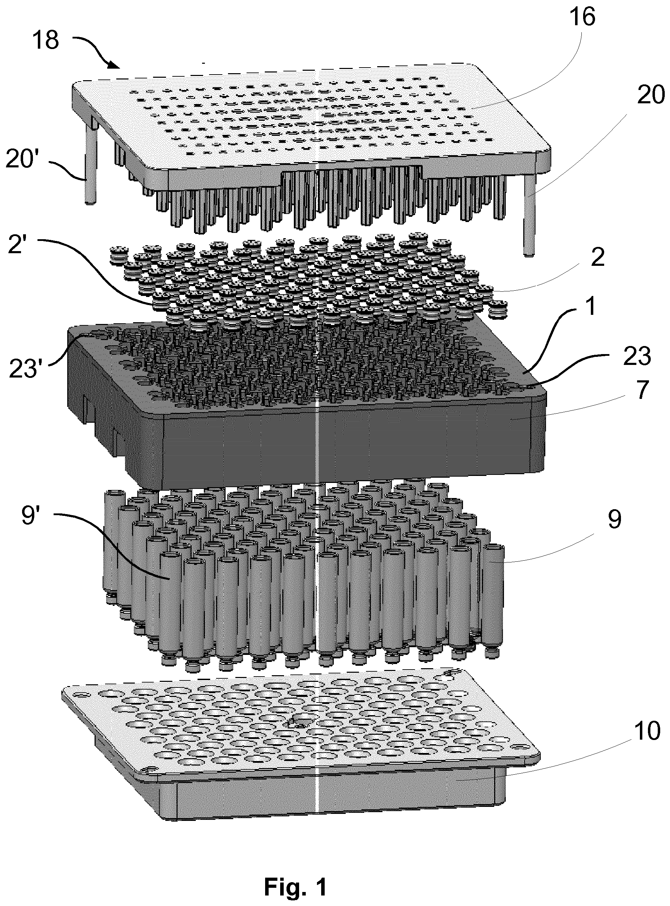

Figur 1 zeigt eine Lyophilisationsanordnung 18, welche auf der vorliegenden Erfindung basiert, in Explosionsansicht. Von oben nach unten sind erkennbar eine Stiftplatte 16 mit Positionierelementen 20,20', welche in dieser Ausführungsform einstückig an der Stiftplatte ausgebildet sind; ein Ensemble von Stopfen 2,2'; eine Halteplatte 1 mit Führungslöchern 23, 23'; ein Stützkragen 7; ein Ensemble von Karpulen 9,9' in bevorzugter Anordnung; sowie eine Sockelplatte 10. In der Explosionsansicht nicht gezeigt sind die optionalen Druckausgleichselemente.

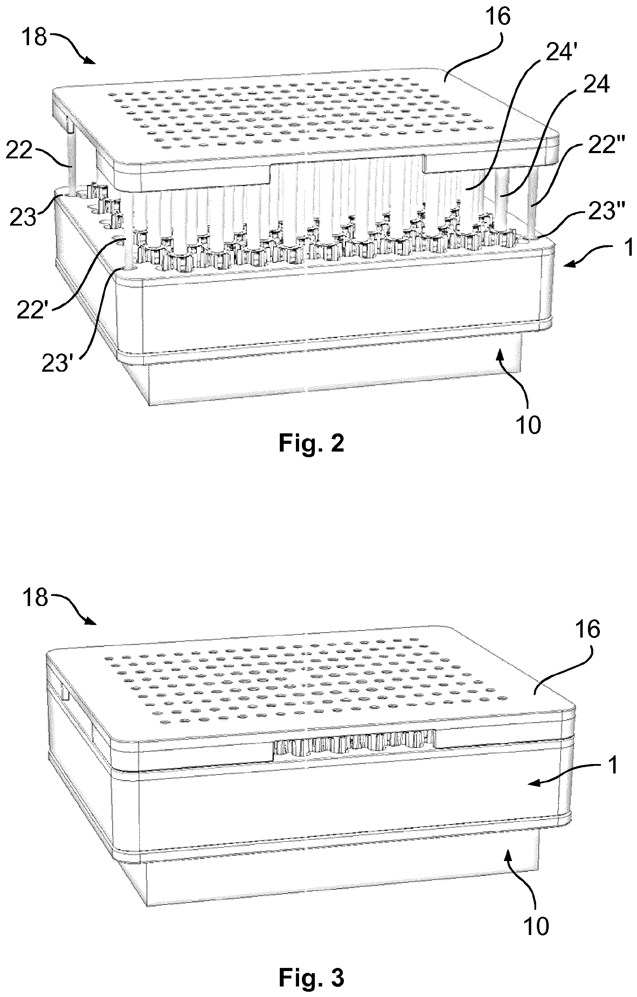

Aus Figur 2 ist ersichtlich, wie die Stiftplatte 16 auf die Halteplatte 1 aufgelegt wird. Die Stiftplatte 16 weist an ihren Ecken Positionierbolzen 22-22" auf, die in Führungslöcher 23-23'' der Halteplatte 1 eingreifen. Die Positionierbolzen greifen ebenfalls in die auf der Oberseite der Sockelplatte eingelassenen Vertiefungen ein. Auch die Halteplatte kann Positionierungsbolzen aufweisen. Dadurch können die Stifte 24, 24' der Stiftplatte 16 relativ zu den Stopfen 2, 2' und relativ zu den Karpulen konzentrisch ausgerichtet werden. Die resultierende Anordnung bildet die Lyophilisationsanordnung 18 des entsprechenden Ausführungsbeispiels in einer ersten Position, jedoch ohne Durckausgleichselemente. Die Anordnung wird in eine in der pharmazeutischen Industrie gebräuchliche Lyophilisationsapparatur eingelegt, um die in den Karpulen 9, 9' vorhandene Lyophilisationslösung einer Gefriertrocknung zu unterziehen.

Die Figur 3 zeigt die Lyophilisationsanordnung 18 in einer zweiten Position, in welcher die Stopfen vollständig in die zugehörigen Karpulen eingebracht sind. Die Stiftplatte 16 wird hierzu auf die Halteplatte 1 heruntergedrückt. Durch das Herunterdrücken der Stiftplatte 16 werden die Stopfen 2, 2' in die Karpulen 9, 9' eingesetzt und innerhalb des länglichen Karpulenkörpers auf eine gewünschte Position gebracht.

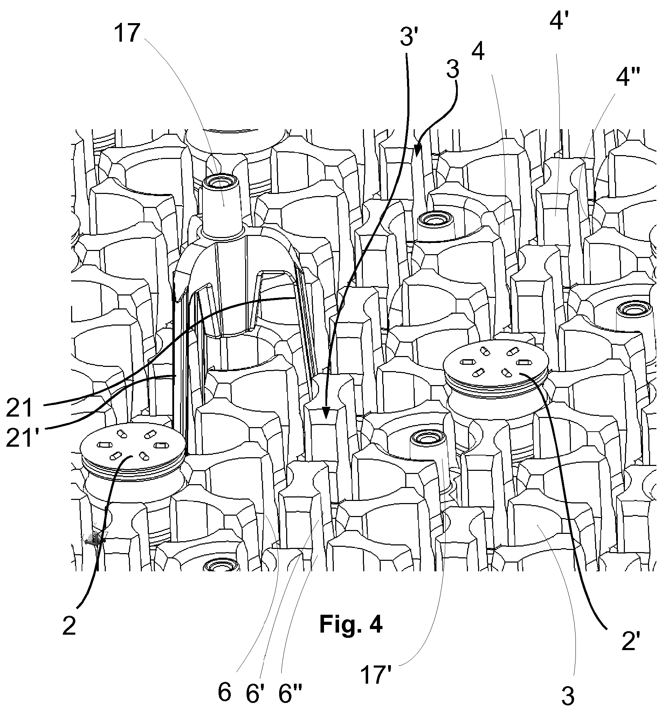

Figur 4 zeigt perspektivisch eine Teilvergrösserung der Halteplatte (dem Gefäss abgewandte Seite) mit Stopfen und Druckausgleichselementen. Es ist zu erkennen, dass die Stopfen 2, 2' jeweils in einer Stopfenaufnahme 3, 3' gehalten sind. Die Stopfenaufnahmen 3, 3' sind in einem Raster angeordnet. Jede einzelne Stopfenaufnahme umfasst sechs Innenwandabschnitte, von welchen vorliegend die Innenwandabschnitte 4 bis 4" gekennzeichnet sind. Zwischen den Innenwandabschnitten 4 bis 4" sind Entgasungsöffnungen angeordnet. Sie sind zur besseren Übersicht an einer anderen Aufnahmeöffnung gekennzeichnet, 6 bis 6". Durch diese Öffnungen 6 bis 6" können aus den Karpulen 9,9' austretende flüchtige Bestandteile während einer Lyophilisation die Halteplatte 1 passieren.

Durch die Druckausgleichselemente 17,17' wird der Innendruck der Karpulen dem Aussendruck angepasst. Während sich das Druckausgleichselement 17' bereits in anwendungsbereiter Position befindet, ist Druckausgleichselement 17 in der Darstellung zwecks Erkennbarkeit etwa zur Hälfte aus seiner anwendungsbereiten Position in der Halteplatte herausgezogen. Das Druckausgleichselement ist in der Darstellung dreibeinig ausgebildet und sitzt während der Anwendung in einer Sattelvertiefung an der Oberfläche der Halteplatte, sodass jedes Bein 21, 21' in eine unterschiedliche Aufnahmeöffnung 3,3' ragt. Nach Entfernen der Karpulen 9, 9' aus der Lyophilisationsanordnung 18 und Herausziehen der Druckausgleichselemente 17, 17' aus den Karpulen 9, 9' sind die Karpulen 9, 9' hermetisch verschlossen.

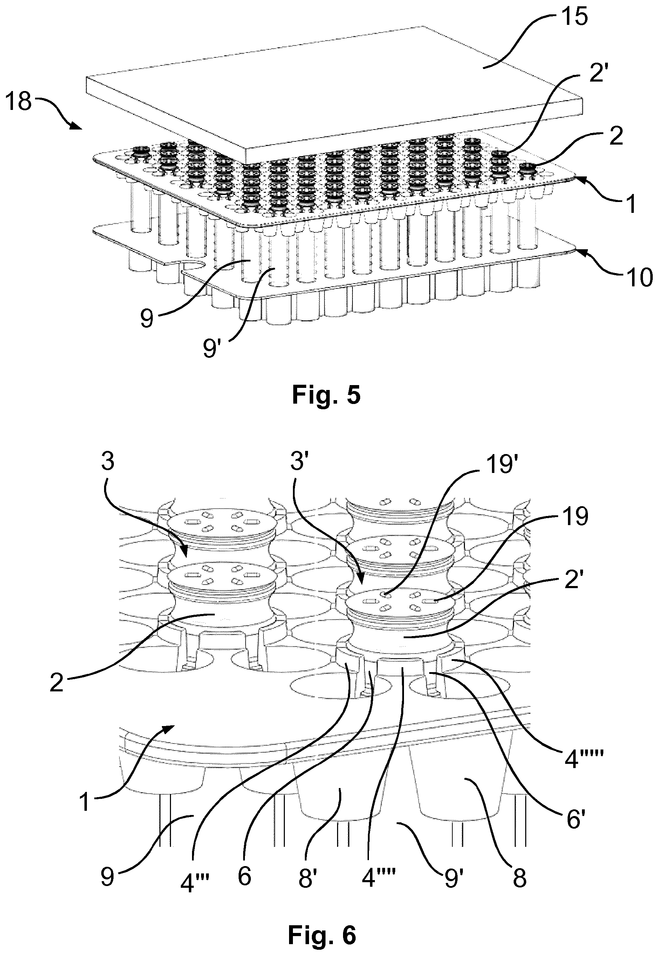

Figur 5 zeigt ein Set von Karpulen 9, 9', die mit ihrem Hals nach unten in eine Sockelplatte eingelegt sind, welche vorliegend als Sockelplatte 10 ausgebildet ist. Auf den Karpulen 9, 9' liegt in dieser Ausführungsform die Halteplatte 1 auf. Durch die Halteplatte 1 sind die Stopfen 2, 2' gehalten. Die vorliegende Abbildung verdeutlicht schematisch die Situation der Lyophilisationsvorrichtung 18 in einer Anordnung mit Beaufschlagplatte 15. Die Beaufschlagplatte 15 ist in der Regel Teil der Lyophilisationsapparatur.

Aus der Teilvergrösserung gemäss Figur 6 sind weitere Einzelheiten zur dieser Ausführungsform der Halteplatte 1 ersichtlich. Erneut sind die Stopfen 2, 2' jeweils in einer Stopfenaufnahme 3, 3' gehalten, welche jeweils sechs Griffbacken 4‴, 4ʺʺ umfasst. Zwischen den Griffbacken 4‴, 4ʺʺ sind Öffnungen angeordnet, welche vorliegend als Schlitze 6, 6' ausgebildet sind. Um eine Stopfenaufnahme 3, 3' ist zudem jeweils eine Zentrierstruktur in Form von sechs Vorsprüngen 8, 8' angeordnet. Die Zentrierstruktur dient dazu, die Halteplatte, welche im vorliegenden Beispiel auf den Karpulen 9, 9' aufliegt, derart zu zentrieren, dass jeweils ein Stopfen 2, 2' konzentrisch auf einer Karpule 9, 9' ausgerichtet ist. Darüber hinaus ist zu erkennen, dass jeder Stopfen auf seiner Oberseite jeweils sechs Vorsprünge 19, 19' aufweist. Diese dienen dazu, ein Festfrieren der Stopfen 2, 2' an der Beaufschlagplatte 15 zu verhindern.

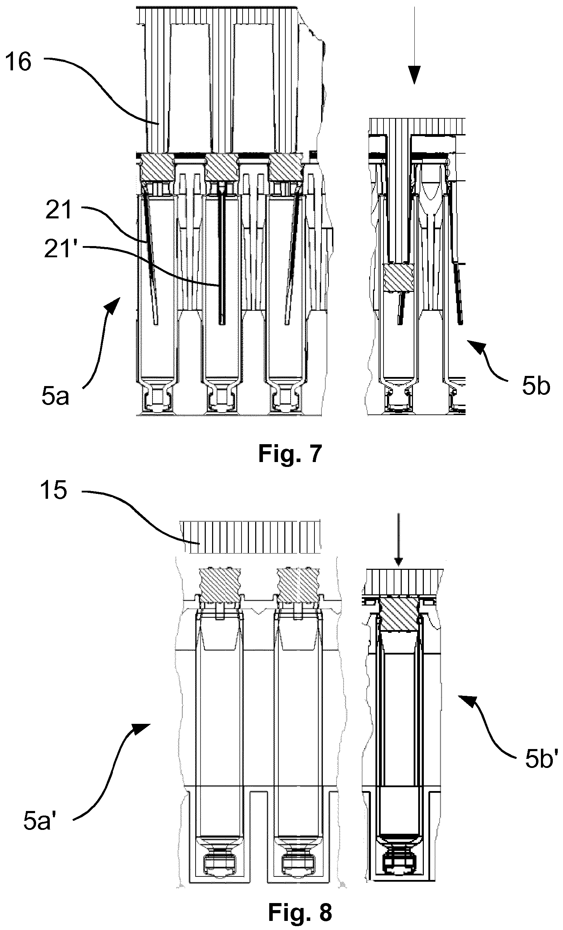

Anhand den nachfolgend diskutierten Figuren 7 und 8 soll ein erfindungsgemässes Verfahren zum Verschieben des Stopfens aus einer ersten Position über der Öffnung 5a,5a' in eine zweite Position, in welcher wenigstens ein Abschnitt des Stopfens die Öffnung des Gefässes verschliesst 5b, 5b' illustriert werden. Dabei zeigt Figur 7 das Ausführungsbeispiel mit Stiftplatte, während Figur 8 das Ausführungsbeispiel mit Beaufschlagplatte erhellt. Alle Situationen zeigen ausschnittsweise den Querschnitt durch eine Reihe von Karpulen in der erfindungsgemässen Vorrichtung. In der ersten Position 5a,5a' werden die Stopfen während des Lyophilisiervorgangs durch Innenwandabschnitte über der Öffnung des Gefässes gehalten, sodass der flüchtige Teil des Karpuleninhalts durch die Schlitze zwischen den Innenwandabschnitten entweichen kann. In der zweiten Position 5b,5b' wurde von oben Druck auf die Stiftplatte 16 respektive die Beaufschlagplatte 15 ausgeübt, sodass der gesamte Stopfen (in 5b) respektive ein Abschnitt des Stopfens (in 5b') die Öffnung der Karpule verschliesst. In Figur 7 ist zudem ersichtlich, wie jeweils ein Bein 21, 21' des Druckausgleichselements zwischen dem Stopfen und der Karpulenwand in das Gefäss hineinragt.

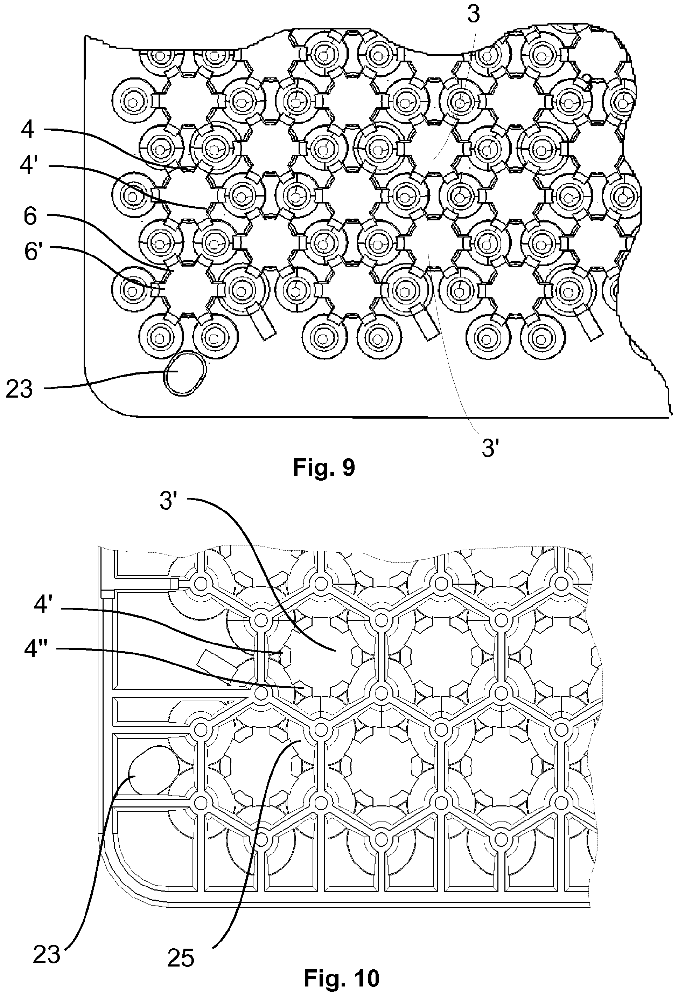

Figur 9 zeigt die Halteplatte der bevorzugten Ausführungsform von Figur 1 in der Draufsicht. Figur 10 zeigt die gleiche Halteplatte in der Ansicht von unten. Sichtbar sind die Aufnahmeöffnungen 3,3' mit den Innenwandabschnitten 4,4".

In diesem Ausführungsbeispiel sind die Strukturen in den Wabenecken 25 als sich von der Oberfläche weg verjüngende Kegelsegmente ausgebildet, aufweisend einen Anzug von ca. 1 bis 2 Grad. Sie dienen so der Zentrierung der Karpulen bezüglich der Stopfen. Die in der Draufsicht, Fig. 9, zwischen den Aufnahmeöffnungen 3,3' sichtbaren Rundstrukturen sind Sattelvertiefungen, in welche die dreibeinig ausgebildeten Druckausgleichselemente 17 (nicht gezeigt) gesetzt werden können, sodass jedes Bein in eine unterschiedliche Aufnahmeöffnung 3, 3' ragt.

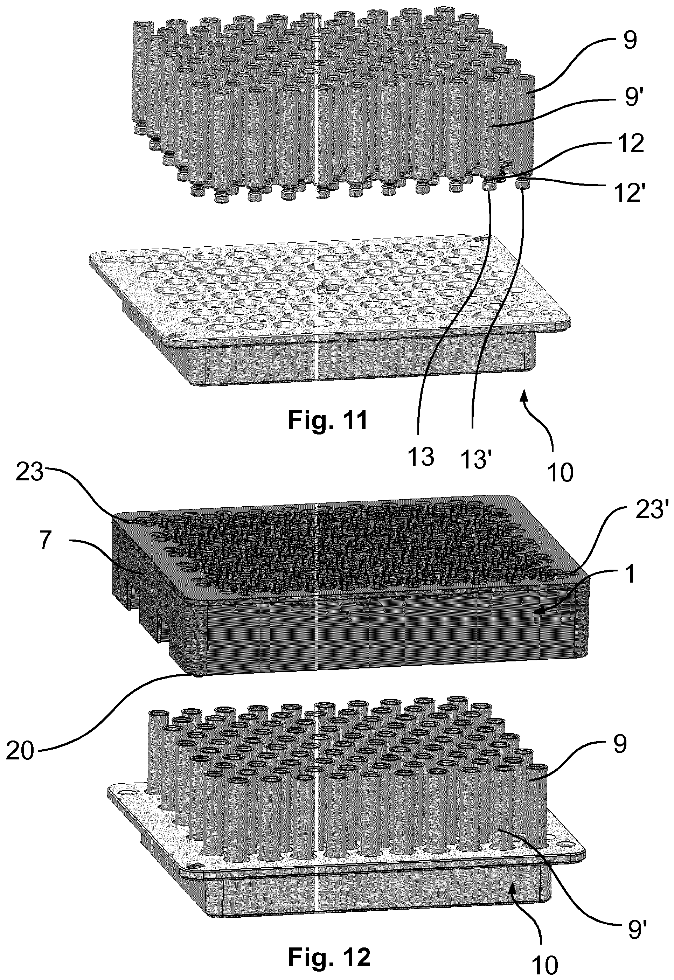

Die Figur 11 zeigt einen ersten Verfahrensschritt, bei dem ein Set von Karpulen 9, 9' mit ihrem Hals 12, 12' nach unten in eine Sockelplatte 10 eingelegt wird. Es ist zu erkennen, dass der Hals 12, 12' der Karpulen 9, 9' jeweils mit einem Septum 13, 13' verschlossen ist. Die als Karpulenhalterung ausgeführte Sockelplatte 10 ist aus einem Metall gefertigt, um einen besseren Wärmeübergang während der Lyophilisation zu ermöglichen und um die zu lyophilisiernde Lösung von Umgebungsstrahlung abzuschirmen. An den Ecken der Halteplatte 10 sind jeweils Langlöcher zur Aufnahme von Zentrierstrukturen 20 bis 20" angeordnet.

Wie in Figur 12 dargestellt ist, wird in einem nächsten Schritt eine Halteplatte 1 auf die Sockelplatte 10 aufgelegt. Die Halteplatte 1 weist an ihren Ecken Führungslöcher 23,23' zur Aufnahme der Zentrierstrukturen auf. Die Halteplatte 1 weist ihrerseits eine Zentrierstruktur 20 zur Anordnung gegenüber der Halteplatte auf. Dadurch ist ein exaktes Zentrieren der Halteplatte 1 relativ zu den Karpulen 9, 9' möglich. Die Halteplatte 1 weist an ihrer Unterseite einen Stützkragen 7 auf, so dass die Halteplatte 1 nicht auf den Karpulen 9, 9', sondern auf der Sockelplatte 10 aufliegt.

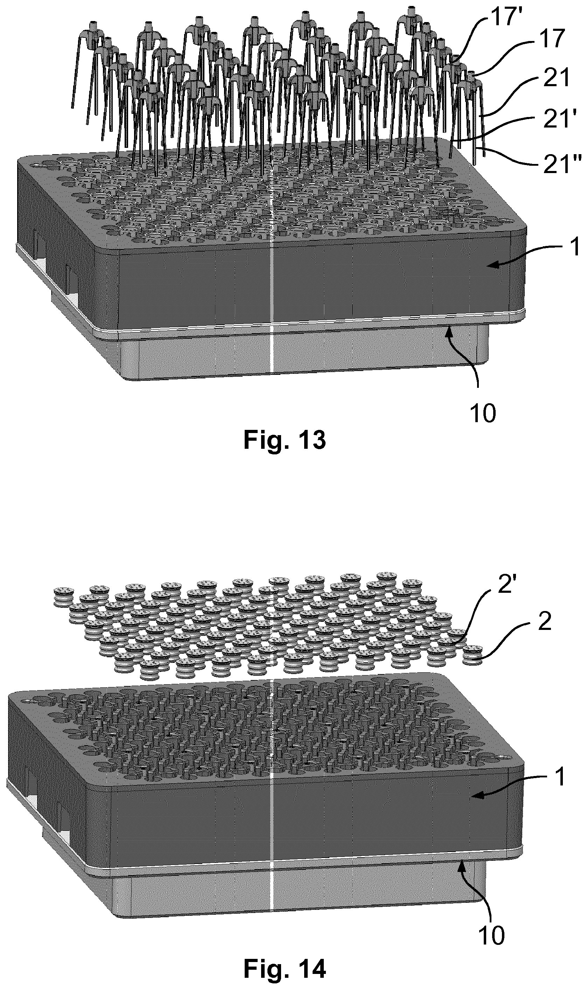

Wie in Figur 13 dargestellt, werden in einem nächsten Schritt Druckausgleichselemente 17, 17' von oben in die Karpulen 9, 9' eingelegt. Ein einzelnes Druckausgleichselement 17 verfügt dabei über drei Beine 21 bis 21", von welchen jedes jeweils in eine andere Karpule 9,9' hineinragen kann. Die Druckausgleichselemente 17,17' sind derart ausgelegt, dass wenn ein Stopfen 2 in eine Karpule 9 eingesetzt wird, eines der Beine 21 zwischen der Karpulenwand und dem Stopfen 2 platziert ist. Da der Stopfen 2 elastisch ist, wird dieser im Bereich des Beins 21 etwas von der Karpulenwand weggedrückt, wodurch Luft oder auch ein anderes Gas zwischen der Karpulenwand und dem Stopfen 2 passieren kann.

Wie aus Figur 14 ersichtlich ist, werden in einem nächsten Schritt die Stopfen 2, 2' in die Stopfenaufnahmen 3,3' der Halteplatte 1 eingelegt. Daraufhin kann der Lyophilisationsvorgang gestartet werden. Optional kann während des Lyophilisationsvorgangs auch bereits die Stiftplatte 16 auf die Stopfen aufgelegt werden, ohne jedoch diese Stopfen wesentlich zu verschieben. Den Abschluss des Verfahrens bildet das Verschliessen der Karpulenöffnungen, wie es aus den Figuren 2,3,7,8 ersichtlich ist.

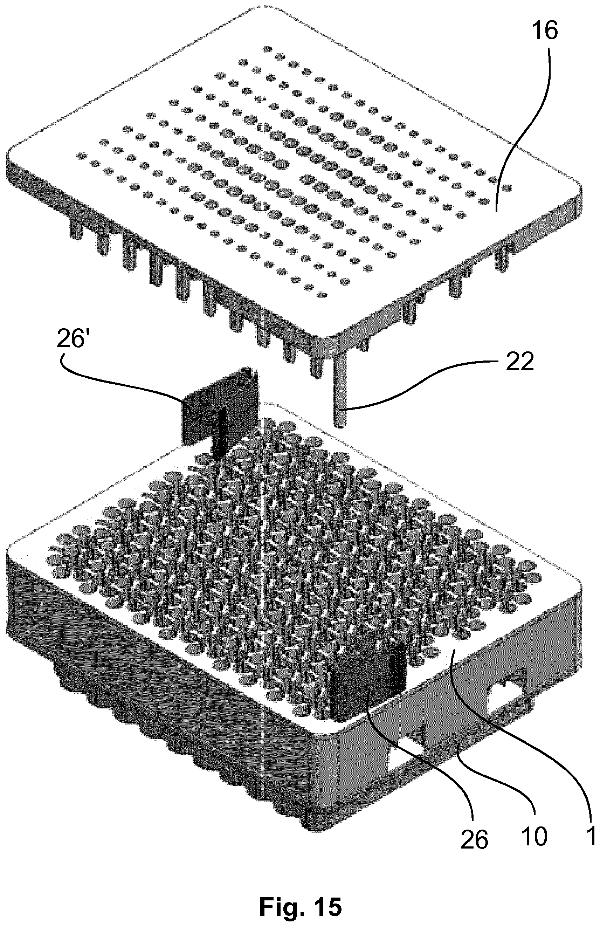

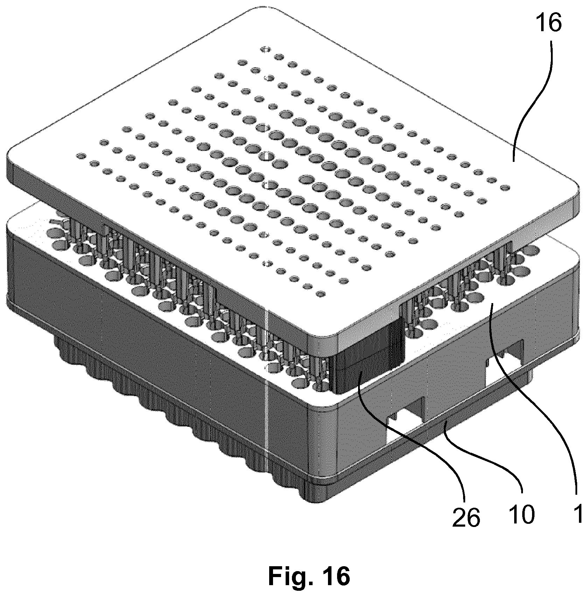

Aus den Figuren 15 und 16 ist ein Set ersichtlich, welches neben der Stopfenhalterung 1 und den Stopfen (im Bild nicht gezeigt) zusätzlich eine Stiftplatte 16, eine Sockelplatte 10 und zwei Abstandhaltemittel 26, 26' enthält. Fig. 15 zeigt die Situation vor dem Assemblieren der Stiftplatte. Fig. 16 zeigt, abgesehen von den fehlenden Stopfen, das fertig assemblierte und verpackungsbereite Set. Gemäss Fig. 15 sind die Abstandhaltemittel 26, 26' als Klemmen ausgebildet und so positioniert, dass sie an zwei Ecken der Vorrichtung einen konstanten Abstand zwischen Stiftplatte und der Stopfenhalterung garantieren. In Fig. 16 umklammern die Klemmen die an der Stiftplatte ausgebildeten Führungsstifte 22, 22'.

Während des Transports verbleiben die Klemmen in der gezeigten, mit der Seitenfläche der Vorrichtung bündigen Position. Das so gesicherte Set ist zum Beispiel stapelbar, ohne dass die Stopfen vorzeitig verschoben würden. Kurz vor oder nach dem Einbringen des Sets in eine Lyophilisierapperatur oder in eine sterile Umgebung werden die Klemmen sodann nach aussen rotiert und lassen sich einfach entfernen.

## Patentansprüche

1. Vorrichtung zum Halten von wenigstens einem Stopfen (2) über der Öffnung jeweils einer Karpule (9) für den Einsatz in einer Lyophilisationsapparatur, umfassend
eine über der Karpule (9) positionierbare Halteplatte (1), welche mehrere Aufnahmeöffnungen (3) für die Aufnahme mehrerer Stopfen, aufweist,
wobei die Aufnahmeöffnungen Innenwandabschnitte (4, 4', 4") aufweisen, welche den Stopfen (2) derart verschiebbar aufnehmen, dass er aus einer ersten Position (5a) über der Öffnung der Karpule (9) in eine zweite Position verschiebbar ist, in welcher wenigstens ein Abschnitt des Stopfens (2) die Öffnung der Karpule (9) verschliesst (5b),
wobei die Halteplatte (1) wenigstens eine Entgasungsöffnung aufweist, durch welche bei in die Aufnahmeöffnung (3) eingesetztem Stopfen (2) ein Gas von der der Karpule (9) zugewandten Seite der Halteplatte auf die von der Karpule (9) abgewandte Seite der Halteplatte strömen kann, wobei die Entgasungsöffnung als Schlitz (6) zwischen wenigstens zwei Innenwandabschnitten (4, 4') ausgebildet ist;
wobei an der Halteplatte auf der der Karpule (9) zugewandten Seite ein um die Halteplatte umlaufender Stützkragen (7) angeordnet ist, an dem die Halteplatte (1) abstützbar ist, insbesondere auf einer Sockelplatte abstützbar ist,
**dadurch gekennzeichnet, dass** die Vorrichtung weiter umfasst
wenigstens ein Druckausgleichselement (17), so beschaffen, dass es mehrere Beine aufweist, von denen jedes gleichzeitig durch die Aufnahmeöffnung (3) hindurch in eine andere Karpule (9) einbringbar ist und zwischen Stopfen (2) und Karpulenwand in die Karpule (9) hineinragen kann.

2. Vorrichtung nach Anspruch 1, wobei die Innenwandabschnitte durch wenigstens zwei Griffbacken (4‴, 4ʺʺ, 4‴ʺ) gebildet werden, zwischen denen der Stopfen (2) haltbar ist.

3. Vorrichtung nach einem der Ansprüche 1 bis 2, wobei die Halteplatte an der der Karpule (9) zugewandten Seite eine Zentrierstruktur zum Zentrieren von zumindest einer Aufnahmeöffnung (3) gegenüber einer Karpule (9) aufweist.

4. Vorrichtung nach einem der Ansprüche 1 bis 3, weiter umfassend eine Stiftplatte (16), so beschaffen, dass sie auf die Halteplatte (1) aufsetzbar ist, sodass auf den wenigstens einen Stopfen (2) jeweils das Ende eines Stifts (24) zu liegen kommt.

5. Vorrichtung nach Anspruch 4, wobei die auf die Halteplatte (1) aufsetzbare Stiftplatte (16) Entgasungsöffnungen aufweist, wobei es bevorzugt ist, dass die Entgasungsöffnungen der Stiftplatte im Zentrum grösser sind als in Randnähe der Stiftplatte.

6. Vorrichtung nach einem der Ansprüche 1 bis 5, weiter umfassend eine Sockelplatte (10) mit Aufnahmeöffnungen für wenigstens eine Karpule.

7. Vorrichtung nach Anspruch 6, wobei die Sockelplatte (10) als insbesondere einstückige Sockelplatte zum Halten von mehreren Karpulen (9, 9') ausgebildet ist und vorzugsweise aus einem Metall gefertigt ist.

8. Vorrichtung nach einem der Ansprüche 6 oder 7, wobei die Sockelplatte (10) wenigstens ein komplementäres Positioniermittel umfasst, mit welchem die Halteplatte (1) und die Sockelplatte (10), und gegebenenfalls zusätzlich die Stiftplatte, gegenseitig positionierbar sind.

9. Set umfassend eine Vorrichtung nach einem der Ansprüche 1 bis 8, umfassend zumindest die Halteplatte (1) und zumindest einen Stopfen (2), wobei in jede Aufnahmeöffnung (3, 3'), der Halteplatte (1) ein Stopfen (2) eingesetzt ist, wobei das Set insbesondere zusätzlich umfasst:
- die Stiftplatte (16), so beschaffen, dass sie auf die Halteplatte (1) aufsetzbar ist, sodass auf den wenigstens einen Stopfen (2) jeweils das Ende eines Stifts (24) zu liegen kommt; und/oder
- die Sockelplatte (10) mit Aufnahmeöffnungen für wenigstens eine Karpule (9).

10. Verfahren zum Befüllen von zumindest einer Karpule (9), insbesondere mehrerer Karpulen (9, 9'), mit einem Lyophilisat, umfassend die folgenden Schritte:
- Bereitstellen der zumindest einen Karpule (9), wobei die Karpule (9) von einer Sockelplatte (10) einer Vorrichtung nach einem der Ansprüche 6 bis 8 gehalten ist;
- Befüllen der zumindest einen Karpule (9) mit einer Lyophilisationslösung;
- Bereitstellen von zumindest einem Stopfen (2), wobei der Stopfen (2) von einer Halteplatte (1) einer Vorrichtung nach einem der Ansprüche 1 bis 3, gehalten ist;
- anschliessendes Positionieren des zumindest einen Stopfens (2) mittels der Halteplatte (1) im Bereich einer Öffnung (11) der Karpule (9);
- Lyophilisieren der Lyophilisationslösung mittels einer Lyophilisationsapparatur, um ein Lyophilisat zu erhalten;
- Einsetzen des zumindest einen Stopfens (2) in die zumindest eine Karpule (9)
- Einsetzen wenigstens eines Druckausgleichselementes (17) einer Vorrichtung nach einem der Ansprüche 1 bis 8 in die wenigstens eine Karpule (9), vor dem Einsetzen des wenigstens einen Stopfens (2) in die Karpule (9);
- Entfernen des Druckausgleichselementes (17) aus der Karpule (9), nach dem Einsetzen des Stopfens (2) in die Karpule (9).

11. Verfahren nach Anspruch 10, wobei das Einsetzen des zumindest einen Stopfens (2) in die zumindest eine Karpule (9) durch Beaufschlagen des Stopfens (2) mit einer Kraft von der der Karpule (9) abgewandten Seite und durch Einschieben wenigstens eines Abschnitts des Stopfens (2) in die Öffnung (11) der Karpule (9) erfolgt.

12. Verfahren nach einem der Ansprüche 10 oder 11, wobei das Einsetzen wenigstens eines Abschnitts des zumindest einen Stopfens (2) in die zumindest eine Karpule (9) bei einem Druck geringer als Atmosphärendruck erfolgt.

13. Verfahren nach Anspruch 12, zusätzlich umfassend den Schritt Belüften der Lyophilisationsapparatur auf Atmosphärendruck, wobei bevorzugt der zumindest eine Stopfen (2) sich beim Belüften der Lyophilisationsapparatur auf Atmosphärendruck entlang der Längsrichtung der Karpule (9) bewegt.

## Claims

1. Device for holding at least one stopper (2) above the opening of a carpule (9) for use in a lyophilization apparatus, comprising
a holding plate (1) that can be positioned above the carpule (9) and which has a plurality of receiving openings (3) for receiving a plurality of stoppers,
the receiving openings having inner wall portions (4, 4', 4") which receive the stopper (2) in such a way that it can be moved from a first position (5a) above the opening of the carpule (9) to a second position in which at least one portion of the stopper (2) closes the opening of the carpule (9) (5b),
the holding plate (1) having at least one degassing opening through which, when the stopper (2) is inserted into the receiving opening (3), a gas can flow from the side of the holding plate facing the carpule (9) to the side of the holding plate facing away from the carpule (9), the degassing opening being designed as a slot (6) between at least two inner wall portions (4, 4');
a support collar (7) being arranged on the side of the holding plate facing the carpule (9), circumferential around the holding plate, on which collar the holding plate (1) can be supported, in particular on a base plate,
**characterized in that** the device further comprises
at least one pressure equalization element (17) designed to have a plurality of legs, each of which can be inserted simultaneously through the receiving opening (3) into another carpule (9) and can project into the carpule (9) between the stopper (2) and the carpule wall.

2. Device according to claim 1, wherein the inner wall portions are formed by at least two gripping jaws (4‴, 4ʺʺ, 4‴ʺ) between which the stopper (2) can be held.

3. Device according to one of claims 1 to 2, wherein the holding plate has a centering structure on the side facing the carpule (9) for centering at least one receiving opening (3) relative to a carpule (9).

4. Device according to any of claims 1 to 3, further comprising a pin plate (16) designed to be placeable on the holding plate (1) so that the end of a pin (24) rests on the at least one stopper (2).

5. Device according to claim 4, wherein the pin plate (16) which can be placed on the holding plate (1) has degassing openings, wherein it is preferred that the degassing openings of the pin plate are larger in the center than near the edge of the pin plate.

6. Device according to any of claims 1 to 5, further comprising a base plate (10) having receiving openings for at least one carpule.

7. Device according to claim 6, wherein the base plate (10) is designed as a particularly one-piece base plate for holding a plurality of carpules (9, 9') and is preferably made of a metal.

8. Device according to one of claims 6 or 7, wherein the base plate (10) comprises at least one complementary positioning means with which the holding plate (1) and the base plate (10), and optionally additionally the pin plate, can be positioned relative to each other.

9. Set comprising a device according to any of claims 1 to 8, comprising at least the holding plate (1) and at least one stopper (2), wherein a stopper (2) is inserted into each receiving opening (3, 3') of the holding plate (1), wherein the set in particular further comprises:
- the pin plate (16), designed to be placeable on the holding plate (1) so that the end of a pin (24) rests on the at least one stopper (2); and/or
- the base plate (10) having receiving openings for at least one carpule (9).

10. Method for filling at least one carpule (9), in particular a plurality of carpules (9, 9'), with a lyophilisate, comprising the following steps:
- providing the at least one carpule (9), wherein the carpule (9) is held by a base plate (10) of a device according to any of claims 6 to 8;
- filling the at least one carpule (9) with a lyophilization solution;
- providing at least one stopper (2), wherein the stopper (2) is held by a holding plate (1) of a device according to any of claims 1 to 3;
- subsequent positioning of the at least one stopper (2) by means of the holding plate (1) in the region of an opening (11) of the carpule (9);
- lyophilizing the lyophilization solution using a lyophilization apparatus to obtain a lyophilisate;
- inserting the at least one stopper (2) into the at least one carpule (9)
- inserting at least one pressure equalization element (17) of a device according to any of claims 1 to 8 into the at least one carpule (9) before inserting the at least one stopper (2) into the carpule (9);
- removing the pressure equalization element (17) from the carpule (9) after inserting the stopper (2) into the carpule (9).

11. Method according to claim 10, wherein the at least one stopper (2) is inserted into the at least one carpule (9) by applying a force to the stopper (2) from the side facing away from the carpule (9) and by sliding at least one portion of the stopper (2) into the opening (11) of the carpule (9).

12. Method according to one of claims 10 or 11, wherein at least one portion of the at least one stopper (2) is inserted into the at least one carpule (9) at a pressure lower than atmospheric pressure.

13. Method according to claim 12, further comprising the step of venting the lyophilization apparatus to atmospheric pressure, wherein preferably the at least one stopper (2) moves along the longitudinal direction of the carpule (9) when the lyophilization apparatus is vented to atmospheric pressure.

## Revendications

1. Dispositif permettant de maintenir au moins un bouchon (2) au-dessus de l'ouverture de respectivement une carpule (9) pour l'utilisation dans un appareil de lyophilisation, comprenant
une plaque de maintien (1) pouvant être positionnée au-dessus de la carpule (9), laquelle plaque de maintien présente plusieurs ouvertures de réception (3) pour la réception de plusieurs bouchons,
dans lequel les ouvertures de réception présentent des sections formant parois intérieures (4, 4', 4") qui reçoivent le bouchon (2) de manière à pouvoir le coulisser de telle sorte qu'il peut coulisser d'une première position (5a) au-dessus de l'ouverture de la carpule (9) à une seconde position dans laquelle au moins une section du bouchon (2) ferme (5b) l'ouverture de la carpule (9),
dans lequel la plaque de maintien (1) présente au moins une ouverture de dégazage à travers laquelle, lorsque le bouchon (2) est inséré dans l'ouverture de réception (3), un gaz peut s'écouler du côté de la plaque de maintien tourné vers la carpule (9) vers le côté de la plaque de maintien opposé à la carpule (9), dans lequel l'ouverture de dégazage est réalisée sous forme de fente (6) entre au moins deux sections formant parois intérieures (4, 4') ;
dans lequel une collerette d'appui (7) entourant la plaque de maintien est disposée sur la plaque de maintien sur le côté tourné vers la carpule (9), sur laquelle collerette la plaque de maintien (1) peut s'appuyer, en particulier peut s'appuyer sur une plaque formant base,
**caractérisé en ce que** le dispositif comprend en outre
au moins un élément d'équilibrage de pression (17), adapté de sorte que celui-ci présente plusieurs jambes, dont chacune peut être introduite simultanément dans une autre carpule (9) à travers l'ouverture de réception (3) et peut pénétrer dans la carpule (9) entre le bouchon (2) et la paroi de carpule.

2. Dispositif selon la revendication 1, dans lequel les sections formant parois intérieures sont formées par au moins deux mâchoires de préhension (4‴, 4ʺʺ, 4‴ʺ) entre lesquelles le bouchon (2) peut être maintenu.

3. Dispositif selon l'une des revendications 1 à 2, dans lequel la plaque de maintien présente, sur le côté tourné vers la carpule (9), une structure de centrage pour le centrage d'au moins une ouverture de réception (3) par rapport à une carpule (9).

4. Dispositif selon l'une des revendications 1 à 3, comprenant en outre une plaque à picots (16), adaptée de sorte qu'elle peut être placée sur la plaque de maintien (1) de sorte que respectivement l'extrémité d'un picot (24) vient se placer sur l'au moins un bouchon (2).

5. Dispositif selon la revendication 4, dans lequel la plaque à picots (16) pouvant être placée sur la plaque de maintien (1) présente des ouvertures de dégazage, dans lequel il est préférable que les ouvertures de dégazage de la plaque à picots soient plus grandes au centre qu'à proximité des bords de la plaque à picots.

6. Dispositif selon l'une des revendications 1 à 5, comprenant en outre une plaque formant base (10) comportant des ouvertures de réception pour au moins une carpule.

7. Dispositif selon la revendication 6, dans lequel la plaque formant base (10) est réalisée sous la forme d'une plaque formant base en particulier d'une seule pièce, pour maintenir plusieurs carpules (9, 9'), et est de préférence fabriquée en un métal.

8. Dispositif selon l'une des revendications 6 ou 7, dans lequel la plaque formant base (10) comprend au moins un moyen de positionnement complémentaire avec lequel la plaque de maintien (1) et la plaque formant base (10), et éventuellement en outre la plaque à picots, peuvent être positionnées mutuellement.

9. Ensemble comprenant un dispositif selon l'une des revendications 1 à 8, comprenant au moins la plaque de maintien (1) et au moins un bouchon (2), dans lequel un bouchon (2) est inséré dans chaque ouverture de réception (3, 3') de la plaque de maintien (1), dans lequel l'ensemble comprend en particulier en outre :
- la plaque à picots (16), adaptée de sorte qu'elle peut être placée sur la plaque de maintien (1), de sorte que respectivement l'extrémité d'un picot (24) vient se placer sur l'au moins un bouchon (2) ; et/ou
- la plaque formant base (10) comportant des ouvertures de réception pour au moins une carpule (9).

10. Procédé pour le remplissage d'au moins une carpule (9), en particulier de plusieurs carpules (9, 9'), avec un lyophilisat, comprenant les étapes suivantes :
- fourniture de l'au moins une carpule (9), dans lequel la carpule (9) est maintenue par une plaque formant base (10) d'un dispositif selon l'une des revendications 6 à 8 ;
- remplissage de l'au moins une carpule (9) avec une solution de lyophilisation ;
- fourniture d'au moins un bouchon (2), dans lequel le bouchon (2) est maintenu par une plaque de maintien (1) d'un dispositif selon l'une des revendications 1 à 3 ;
- positionnement subséquent de l'au moins un bouchon (2) au moyen de la plaque de maintien (1) dans la zone d'une ouverture (11) de la carpule (9) ;
- lyophilisation de la solution de lyophilisation à l'aide d'un appareil de lyophilisation afin d'obtenir un lyophilisat ;
- insertion de l'au moins un bouchon (2) dans l'au moins une carpule (9)
- insertion d'au moins un élément d'équilibrage de pression (17) d'un dispositif selon l'une des revendications 1 à 8 dans l'au moins une carpule (9), avant l'insertion de l'au moins un bouchon (2) dans la carpule (9) ;
- retrait de l'élément d'équilibrage de pression (17) de la carpule (9), après l'insertion du bouchon (2) dans la carpule (9).

11. Procédé selon la revendication 10, dans lequel l'insertion de l'au moins un bouchon (2) dans l'au moins une carpule (9) est réalisée en appliquant une force au bouchon (2) depuis le côté opposé à la carpule (9) et en introduisant au moins une section du bouchon (2) dans l'ouverture (11) de la carpule (9).

12. Procédé selon l'une des revendications 10 ou 11, dans lequel l'insertion d'au moins une section de l'au moins un bouchon (2) dans l'au moins une carpule (9) est réalisée à une pression inférieure à la pression atmosphérique.

13. Procédé selon la revendication 12, comprenant en outre l'étape consistant à aérer l'appareil de lyophilisation à la pression atmosphérique, dans lequel de préférence l'au moins un bouchon (2) se déplace le long de la direction longitudinale de la carpule (9) lors de l'aération de l'appareil de lyophilisation à la pression atmosphérique.
